(19) Europäisches Patentamt
European Patent Office
Office européen des brevets

(11) Publication number : **0 330 511 B1**

(12) # EUROPEAN PATENT SPECIFICATION

(45) Date of publication of patent specification : **12.08.92 Bulletin 92/33**

(51) Int. Cl.$^5$ : **A61K 31/557,** // C07C405/00, C08B37/16

(21) Application number : **89301888.7**

(22) Date of filing : **24.02.89**

(54) **Stabilization of 13,14-dihydro-15-ketoprostaglandins.**

The file contains technical information submitted after the application was filed and not included in this specification

(30) Priority : **26.02.88 JP 45622/88**

(43) Date of publication of application : **30.08.89 Bulletin 89/35**

(45) Publication of the grant of the patent : **12.08.92 Bulletin 92/33**

(84) Designated Contracting States : **AT BE CH DE ES FR GB GR IT LI LU NL SE**

(56) References cited :
**EP-A- 0 281 239**
**EP-A- 0 284 180**
**EP-A- 0 289 349**
**EP-A- 0 293 537**
**CHEMICAL ABSTRACTS, vol. 99, no. 16, October 1983, page 344, abstract no. 128179b, Columbus, Ohio, US; K. UEKAMA:"Pharmaceutical application of cyclodextrins", & DENPUN KAGAKU 1983, 30(2), 247-54**

(56) References cited :
**CHEMICAL ABSTRACTS, vol. 102, 11th February 1985, part 6, page 315, abstract no. 50797k, Columbus, Ohio, US; F. HIRAYAMA etal.: "Improving the aqueous stability of prostaglandin E2 and prostaglandin A2 by inclusion complexation withmethylated-beta-cyclodextrins", & CHEM. PHARM. BULL. 1984, 32(10), 4237-40**
**CHEMICAL ABSTRACTS, vol. 106, 1st June 1987, part 22, page 402, abstract no. 182553v, Columbus, Ohio, US; F. HIRAYAMA et al.:"Improvement of chemical instability of prostacyclin in aqueous solution by complexation with methylated cyclodextrins", & INT.J. PHARM. 1987, 35(3), 193-9**
**DATABASE WPIL, no. 84, abstract no. 52386, Derwent Publications; & JP-A-59 010 525 (TEISAN SEIYAKU K.K.)**
**Hirayama et al 1984 Chem. Pharm. Bull. 32, 4237-40, JP 10 525/1984**

(73) Proprietor : **Kabushiki Kaisha Ueno Seiyaku Oyo Kenkyujo**
**4-8, 2-chome, Koraibashi Chuo-ku**
**Osaka-shi Osaka-fu (JP)**

(72) Inventor : **Ueno, Ryuji**
**7-29, Misaku-cho**
**Nishinomiya-shi Hyogo-ken (JP)**
Inventor : **Kuno, Sachiko**
**11-4-603, 1-chome Sakasedai**
**Takarazuka-shi Hyogo-Ken (JP)**

(74) Representative : **Atkinson, Peter Birch et al**
**Marks & Clerk Suite 301 Sunlight House Quay Street**
**Manchester M3 3JY (GB)**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**EP 0 330 511 B1**

Jouve, 18, rue Saint-Denis, 75001 PARIS

## Description

The present invention relates to the stabilization of 13,14-dihydro-15-ketoprostaglandins, which find various applications in the medical field.

Prostaglandins (hereinafter, referred to as PG) are members of a class of organic carboxylic acids that are contained in human and most other mammalian tissues or organs and that exhibit a wide range of physiological activities. Naturally occurring prostagladins possess as a common structural feature the prostanoic acid skeleton:

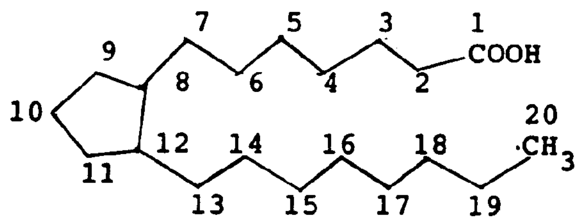

(A)

while some of synthetic analogues have somewhat modified skeletons. The natural prostaglandins are classified based on the structual feature of five-membered cycle moiety as:

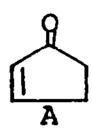
A ,
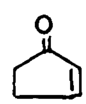
B ,
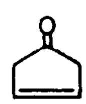
C ,
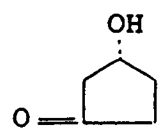
D ,
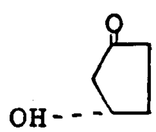
E ,
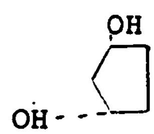
F ,
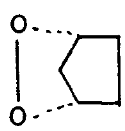
G,H ,
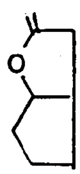
I ,
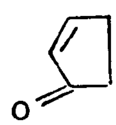
J ,

and also on the presence or absence of unsaturation and/or oxidation in the chain moiety as:

Subscript 1 - - - 15-OH

Subscript 2 - - - 5,6-unsaturated-15-OH

Subscript 3 - - - 5,6- and 17,18-diunsaturated-15-OH.

All the natural prostaglandins are 13,14-unsaturated.

However, the presence of some 13,14-saturated prostaglandins, such as 13,14-dihydro-15-keto-$PGD_2$, 13,14-dihydro-15-keto-$PGE_2$ and 13, 14-dihydro-15-keto-$PGF_{2\alpha}$, are known as the metabolites which have been believed to have no physiological activity.

While the prostaglandins have a wide range of physiological activities and are useful as medicaments based on their respective activities, they have a common fault that they are generally unstable. Various attempts have been made to improve the stability of prostaglandins.

For example, formation of inclusion compounds of PGs or alkyl ester thereof with cyclodextrin (hereinafter, referred to as CD) was disclosed in Japanese Patent Publication No. 3362/1975. Injectable preparations obtained by lyophilyzing PGs or analogues thereof and CD was disclosed in Japanese Patent Publication No. 43569/1979. Injectable preparation obtained by lyophilizing PGE or analogues thereof, CD and ascorbic acid or citric acid was disclosed in Japanese Patent Publication No. 43570/1979. Formation of inclusion compounds of $PGF_{2\alpha}$ analogues with CD was disclosed in Japanese Patent Publication No. 24369/1986. Etherized CDs

could be used in the same manner as CD itself for stabilizing PGE as disclosed in Japanese Patent Publication (unexamined) No. 10525/1984.

The inventors discovered that the 13,14-dihydro-15-keto-PGs have certain physiological activities, contrary to the traditional knowledge that they have no such activities (EP-A 0,284,180, EP-A 0,281,239, EP-A 0,289,349 and EP-A 0,292,177). Attempts have been made, in turn, to stabilize the 13,14-dihydro-15-keto-PGs by means of CD, which, however, have come out to be a failure, confirming that they cannot be stabilized by CD as the result of repeated experiments (unpublished). This means that the stabilization of 13,14-saturated-15-keto-PGs cannot be predicted from the stabilization of 13,14-unsaturated-15-keto-PGs.

In the first aspect, the present invention provides a stabilized 13,14-dihydro-15-ketoprostaglandin composition comprising an intimate mixture of

a) a therapeutically effective amount of at least one compound selected from 13,14-dihydro-15-ketoprostaglandins and

b) at least one compound selected from pharmaceutically acceptable etherized cyclodextrins characterised in that the ratio by weight of component (a) to component (b) is between 1:5 and 1:200

In the second aspect, the present invention provides a method of preparing a stabilized 13,14-dihydro-15-ketoprostaglandin composition which comprises intimately mixing

a) a therapeutically effective amount of at least one

b) at least one compound selected from pharmaceutically acceptable etherized cyclodextrins characterised in that the ratio by weight of component (a) to component (b) is between 1:5 and 1:200.

In the third aspect, the present invention provides a method of stablizing 13,14-dihydro-15-ketoprostaglandins which comprises contacting (A) 13,14-dihydro-15-ketoprostaglandin with (B) etherized cyclodextrin in a solvent capable of at least partly dissolving at least one of (A) and (B).

In the fourth aspect, the present invention provides a stabilizer for 13,14-dihydro-15-ketoprostaglandins comprising etherized cyclodextrin.

According to the invention, it has now be discovered that etherized CDs are useful for stabilization and solubilization of 13,14-dihydro-15-keto-PGs, after exhausting testing of various compounds. It is very surprising that etherized CDs are helpful in the case where CD is not helpful, in view of the close similarity of chemical structure between etherized and unetherized cyclodextrins. Etherized CDs appear to form adducts with 13,14-dihydro-15- keto-PGs ant are presumed to form inclusion compounds analogously to CD.

Nomenclature of 13,14-dihydro-15-keto-PGs herein uses the numbering system of prostanoic acid represented in the formula (A) shown above.

While the formula (A) shows a basic skeleton having twenty carbon atoms, the 13,14-dihydro-15-keto-PGs used in the invention are not limited to those having the same number of carbon atoms. The carbon atoms in the Formula (A) are numbered 2 to 7 on the alpha-chain starting from the alpha-carbon adjacent to the carboxylic carbon which is numbered 1 and towards the five-membered ring, 8 to 12 on the said ring starting from the carbon on which the alpha-chain is attached, and 13 to 20 on the omega-chain starting from the carbon adjacent to the ring. When number of carbon atoms is decreased in the alpha-chain, the number is deleted in order starting from 2-position and when number of carbon atoms is increased in the alpha-chain, compounds are named as substituted derivatives having respective substituents at 1-position in place of carboxyl group (C-1).

Within the 13,14-dihydro-15-ketoprostaglandins as described under item a) above, preferred compounds are those represented by the formula (I):

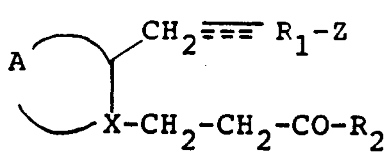

(I)

wherein the group:

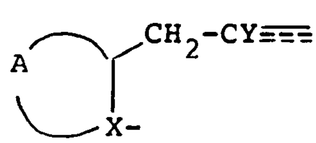

is a radical selected from the following formulae:

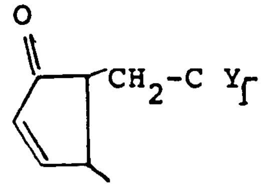
(i)

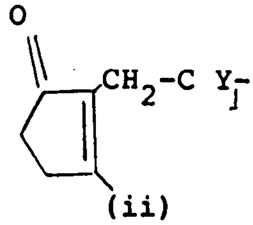
(ii)

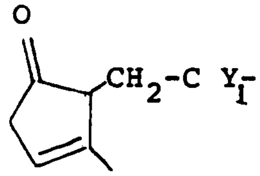
(iii)

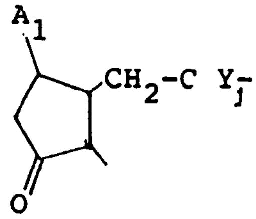
(iv)

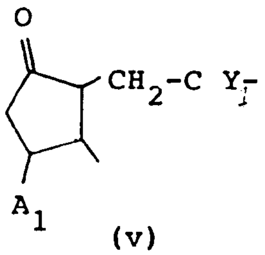
(v)

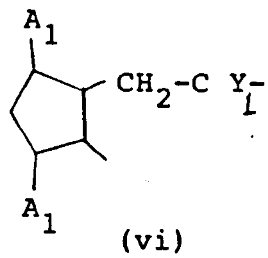
(vi)

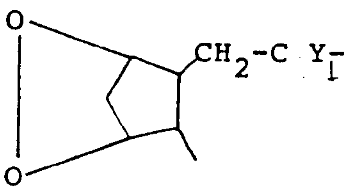
(vii)

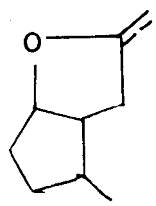
(viii)

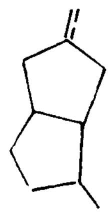
(ix)

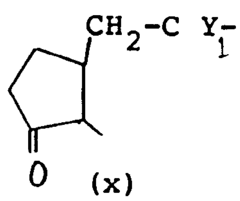
(x)

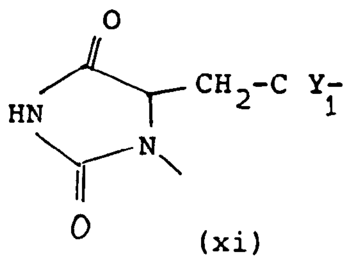
(xi)

$A_1$ is hydroxy, lower alkyl or hydroxy(lower)alkyl,
$Y_1$ is 0, 1 or 2 hydrogen atoms or oxo,
Z is hydroxymethylcarbonyl, carboxy or a functional derivative of carboxy,
$R_1$ is saturated or unsaturated lower aliphatic hydrocarbon residue,
$R_2$ is saturated or unsaturated lower aliphatic hydrocarbon residue which is unsubstituted or substituted with at least one substituent selected from hydroxy, halo, lower-alkoxyphenyl and phenoxy,
the symbol of a line and a dotted line is single bond or double bond, and the symbol of a line and two dotted line is single bond, double bond or triple bond, or a pharmaceutically acceptable salt thereof.

As used herein, the term "lower" is intended to include a group having 1 to 6 carbon atoms unless otherwise specified.

The term "lower" as a group or a moiety in hydroxy(lower)alkyl includes saturated and straight or branched chain hydrocarbon radicals containing 1 to 6, preferably 1 to 5 and more preferably 1 to 4 carbon atoms, such as methyl, ethyl, propyl, isopropyl, butyl, isobutyl, t-butyl, pentyl and hexyl.

The term "lower alkoxy" as a moiety in lower-alkoxy-phenyl refers to the group lower-alkyl-O-phenyl wherein lower alkyl is as defined above.

The term "halo" as a radical denotes fluoro, chloro, bromo and iodo.

The term "functional derivative" of the carboxy includes esters and amides which are used as protective group for carboxy group. Examples of the esters are aliphatic esters, for example, $C_{1-6}$ alkyl ester such as methyl ester, ethyl ester, propyl ester, isopropyl ester, butyl ester, isobutyl ester, t-butyl ester, pentyl ester and 1-cyclopropylethyl ester., lower alkenyl ester such as vinyl ester and allyl ester., lower alkynyl ester such as ethynyl ester and propynyl ester., lower alkoxy(lower)alkyl ester such as methoxymethyl ester and 1-methoxyethyl ester., and aromatic esters, for example, optionally substituted aryl ester such as phenyl ester, tolyl ester, t-butylphenyl ester, salicyl ester, 3,4-di- methoxyphenyl, etc., aryl(lower)alkyl ester such as benzyl ester, trityl ester and benzhydryl ester ester. Examples of the amides are lower alkyl amides such as methylamide, ethylamide, etc., and lower alkylsulfonylamide such as methanesulfonylamide and ethanesulfonylamide.

The term "saturated or unsaturated lower aliphatic hydrocarbon residue" include straight or branched chain aliphatic hydrocarbon residue which may have at least one double or triple bond and up to 6 carbon atoms in the principal chain and up to 3 carbon atoms in any side chain. Such residue may be partly or completely cyclic. Examples of preferred residues for $R_1$ are $-(CH_2)_4-$, $-(CH_2)_2CH=CH-$, $-(CH_2)_2CH(CH_3)CH_2-$, $=CH(CH_2)_3-$, $-(CH_2)_2-CH=CH-$, $-CH=CH-(CH_2)_4$, etc., and examples of preferred residues for $R_2$ are $-(CH_2)_5-$, $-(CH_2)_6-$, $-(CH_2)_7-$, $-(CH_2)_8-$, $-(CH_2)_9-$, $-(CH_2)_3CH(CH_3)CH_2-$, $-C(CH_3)_2(CH_2)_4-$, $-CH_2CH(CH_3)(CH_2)_4-$, $-CH_2CH(CH_3)(CH_2)_5-$, $-C(CH_3)(OCH_3)(CH_2)_4-$, $-C(CH_3)_2CH_2O(CH_2)_2$, $-C(CH_3)_2CH_2O(CH_2)_3-$, cyclopentyl, cyclohexyl, $-CH_2CH(CH_3)(CH_2)_2CH=CH(CH_3)CH_2-$, $-CH(CH_3)CH_2C\equiv CCH_2-$, phenyl, phenoxy, etc.

The configuration of the ring and α- and/or omega chain in the above formula (I) may be the same as or different from that in the natural prostaglandins. However, the present invention also include a mixture of a compound having natural configuration and that of unnatural configuration.

Suitable "pharmaceutically acceptable salt" includes conventional non-toxic salt, and may be a salt with an inorganic base, for example a metal salt such as an alkali metal salt (e.g. sodium salt or potassium salt) and an alkaline earth metal salt (e.g. calcium salt or magnesium salt), ammonium salt, a salt with an organic base, for example, an amine salt (e.g. trimethylamine salt, triethylamine salt, cyclohexylamine salt, benzylamine salt, ethylenediamine salt, ethanolamine salt, diethanolamine salt, triethanolamine salt, tris(hydroxymethylamino)methane salt, procaine salt and caffeine salt), a basic amino acid salt (e.g. arginine salt or lysine salt). These salts can be prepared by the conventional process, for example from the corresponding acid and base or by salt interchange.

The above compounds of the formula (I) include, for example,
13,14-dihydro-15-keto-$PGA_1$s, 13,14-dihydro-15-keto-$PGA_2$s,
13,14-dihydro-15-keto-$PGA_3$s, 13,14-dihydro-15-keto-$PGB_1$s,
13,14-dihydro-15-keto-$PGB_2$s, 13,14-dihydro-15-keto-$PGB_3$s,
13,14-dihydro-15-keto-$PGC_1$s, 13,14-dihydro-15-keto-$PGC_2$s,
13,14-dihydro-15-keto-$PGC_3$s, 13,14-dihydro-15-keto-$PGD_1$s,
13,14-dihydro-15-keto-$PGD_2$s, 13,14-dihydro-15-keto-$PGD_3$s,
13,14-dihydro-15-keto-$PGE_1$s, 13,14-dihydro-15-keto-$PGE_2$s,
13,14-dihydro-15-keto-$PGE_3$s, 13,14-dihydro-15-keto-$PGF_1$s,
13,14-dihydro-15-keto-$PGF_2$s, 13,14-dihydro-15-keto-$PGF_3$s,
13,14-dihydro-15-keto-$PGI_1$s, 13,14-dihydro-15-keto-$PGI_2$s,
13,14-dihydro-15-keto-$PGI_3$s, 13,14-dihydro-15-keto-$PGJ_1$s,
13,14-dihydro-15-keto-$PGJ_2$s, and
13,14-dihydro-15-keto-$PGJ_3$s.

Typical examples of the above compounds of the formula (I) include:

(1) 13,14-dihydro-15-keto-$PGA_1$ methyl ester
(2) 13,14-dihydro-15-keto-$PGA_1$ isopropyl ester
(3) 13,14-dihydro-15-keto-$PGA_2$ methyl ester
(4) 13,14-dihydro-15-keto-$PGA_2$ isopropyl ester
(5) 13,14-dihydro-15-keto-20-ethyl-$PGA_1$ methyl ester
(6) 13,14-dihydro-15-keto-20-ethyl-$PGA_1$ isopropyl ester
(7) 13,14-dihydro-15-keto-20-ethyl-$PGA_2$ methyl ester
(8) 13,14-dihydro-15-keto-20-ethyl-$PGA_2$ isopropyl ester
(9) 13,14-dihydro-15-keto-$PGA_2$
(10) 13,14-dihydro-15-keto-$PGB_1$ methyl ester
(11) 13,14-dihydro-15-keto-$PGB_1$ isopropyl ester
(12) 13,14-dihydro-15-keto-$PGB_2$ methyl ester

(13) 13,14-dihydro-15-keto-$PGB_2$ isopropyl ester
(14) 13,14-dihydro-15-keto-20-ethyl-$PGB_1$ methyl ester
(15) 13,14-dihydro-15-keto-20-ethyl-$PGB_1$ isopropyl ester
(16) 13,14-dihydro-15-keto-20-ethyl-$PGB_2$ methyl ester
(17) 13,14-dihydro-15-keto-20-ethyl-$PGB_2$ isopropyl ester
(18) 13,14-dihydro-15-keto-$PGB_2$
(19) 13,14-dihydro-15-keto-$PGC_1$ methyl ester
(20) 13,14-dihydro-15-keto-$PGC_1$ isopropyl ester
(21) 13,14-dihydro-15-keto-$PGC_2$ methyl ester
(22) 13,14-dihydro-15-keto-$PGC_2$ isopropyl ester
(23) 13,14-dihydro-15-keto-20-ethyl-$PGC_1$ methyl ester
(24) 13,14-dihydro-15-keto-20-ethyl-$PGC_1$ isopropyl ester
(25) 13,14-dihydro-15-keto-20-ethyl-$PGC_2$ methyl ester
(26) 13,14-dihydro-15-keto-20-ethyl-$PGC_2$ isopropyl ester
(27) 13,14-dihydro-15-keto-$PGC_2$
(28) 13,14-dihydro-15-keto-$PGD_1$ methyl ester
(29) 13,14-dihydro-15-keto-$PGD_1$ ethyl ester
(30) 13,14-dihydro-15-keto-$PGD_2$ ethyl ester
(31) 13,14-dihydro-15-keto-$PGD_2$ buthyl ester
(32) 13,14-dihydro-15-keto-5,6-dehydro-$PGDA_2$ methyl ester
(33) 13,14-dihydro-15-keto-5,6-dehydro-9 -hydroxy-$PGD_2$
(34) 13,14-dihydro-15-keto-5,6-dehydro-9 -hydroxy-$PGD_2$ methyl ester
(35) 13,14-dihydro-15-keto-16R,S-fluoro-$PGD_2$ methyl ester
(36) 13,14-dihydro-15-keto-16,16-dimethyl-$PGD_2$ methyl ester
(37) 13,14-dihydro-15-keto-19-methyl-$PGD_2$ methyl ester
(38) 13,14-dihydro-15-keto-20-methoxy-$PGD_2$
(39) 13,14-dihydro-15-keto-20-methoxy-$PGD_2$ butyl ester
(40) 13,14-dihydro-15-keto-16R,S-methyl-20-methoxy-$PGD_2$ methyl ester
(41) 13,14-dihydro-15-keto-20-ethyl-$PGD_1$ methyl ester
(42) 13,14-dihydro-15-keto-20-ethyl-$PGD_1$ ethyl ester
(43) 13,14-dihydro-15-keto-20-ethyl-$PGD_2$ methyl ester
(44) 13,14-dihydro-15-keto-20-ethyl-$PGD_2$ ethyl ester
(45) 13,14-dihydro-15-keto-20-methoxyethyl-$PGD_2$ methyl ester
(46) 13,14-dihydro-15-keto-$PGD_2$
(47) 13,14-dihydro-15-keto-$PGE_1$ ethyl ester
(48) 13,14-dihydro-6,15-diketo-$PGE_1$ ethyl ester
(49) 13,14-dihydro-6,15-diketo-$PGE_1$ butyl ester
(50) ±13,14-dihydro-6,15-diketo-$PGE_1$ ethyl ester
(51) 13,14-dihydro-6,15-diketo-11-dehydroxy-11R-methyl-$PGE_1$ ethyl ester
(52) 13,14-dihydro-6,15-diketo-16R,S-fluoro-11-dehydroxy-11R-methyl-$PGE_1$ ethyl ester
(53) 13,14-dihydro-6,15-diketo-16,16-dimethyl-$PGE_1$ ethyl ester
(54) 13,14-dihydro-6,15-diketo-19-methyl-$PGE_1$ methyl ester
(55) 13,14-dihydro-6,15-diketo-11-dehydroxy-11R-hydroxymethyl-$PGE_1$ ethyl ester
(56) 13,14-dihydro-15-keto-$PGE_2$
(57) 13,14-dihydro-15-keto-$PGE_2$ methyl ester
(58) 13,14-dihydro-15-keto-$PGE_2$ isopropyl ester
(59) 13,14-dihydro-15-keto- $\Delta^2$-$PGE_2$ methyl ester
(60) 13,14-dihydro-15-keto-16R,S-fluoro-$PGE_2$ ethyl ester
(61) 13,14-dihydro-15-keto-3,16-dimethyl-$PGE_2$ methyl ester
(62) 13,14-dihydro-15-keto-16R,S-hydroxy-$PGE_2$ ethyl ester
(63) 13,14-dihydro-15-keto-19-methyl-$PGE_2$ ethyl ester
(64) 13,14-dihydro-15-keto-20-methoxy-$PGE_2$ methyl ester
(65) 13,14-dihydro-15-keto-20-methoxy-$\Delta^2$-$PGE_2$ methyl ester
(66) 13,14-dihydro-15-keto-16,16-dimethyl-20-methoxy-$PGE_2$ methyl ester
(67) 13,14-dihydro-15-keto-20-ethyl-$PGE_1$ methyl ester
(68) 13,14-dihydro-6,15-diketo-20-ethyl-$PGE_1$ ethyl ester
(69) 13,14-dihydro-6,15-diketo-20-ethyl-$PGE_1$ methyl ester
(70) 13,14-dihydro-15-keto-20-ethyl-$PGE_2$ methyl ester

(71) 13,14-dihydro-15-keto-20-ethyl-$PGE_2$ ethyl ester
(72) 13,14-dihydro-15-keto-20-n-propyl-$PGE_2$ methyl ester
(73) 13,14-dihydro-15-keto-20-ethyl-11-dehydroxy-11R-methyl-$PGE_2$ methyl ester
(74) 13,14-dihydro-15-keto-$PGE_2$
(75) 13,14-dihydro-15-keto-$PGF_{1\alpha}$ ethyl ester
(76) 13,14-dihydro-15-keto-$PGF_{2\alpha}$ methyl ester
(77) 13,14-dihydro-15-keto-$PGF_{2\alpha}$ ethyl ester
(78) 13,14-dihydro-15-keto-9 -hydroxy-$PGF_{2\alpha}$ methyl ester
(79) 13,14-dihydro-15-keto-16R,S-fluoro-$PGF_{1\alpha}$
(80) 13,14-dihydro-15-keto-16R,S-fluoro-$PGF_{2\alpha}$
(81) 13,14-dihydro-15-keto-16R,S-fluoro-11-dehydroxy-11R-methyl-$PGF_{2\alpha}$ methyl ester
(82) 13,14-dihydro-15-keto-16,16-dimethyl-$PGF_{2\alpha}$ ethyl ester
(83) 13,14-dihydro-15-keto-17S-methyl-$PGF_{2\alpha}$ ethyl ester
(84) 13,14-dihydro-15-keto-20-ethyl-$PGF_{1\alpha}$ methyl ester
(85) 13,14-dihydro-15-keto-20-ethyl-$PGF_{2\alpha}$
(86) 13,14-dihydro-15-keto-20-ethyl-$PGF_{2\alpha}$ methyl ester
(87) 13,14-dihydro-15-keto-20-ethyl-$PGF_{2\alpha}$ ethyl ester
(88) 13,14-dihydro-15-keto-20-ethyl-$PGF_{2\alpha}$ isopropyl ester
(89) 13,14-dihydro-15-keto-20-ethyl-$PGF_{2\alpha}$ butyl ester
(90) 13,14-dihydro-15-keto-20-methyl-$PGF_{2\alpha}$ methyl ester
(91) 13,14-dihydro-15-keto-20-n-propyl-$PGF_{2\alpha}$ methyl ester
(92) 13,14-dihydro-15-keto-20-n-butyl-$PGF_{2\alpha}$ methyl ester
(93) 13,14-dihydro-15-keto-20-ethyl-16R,S-fluoro-$PGF_{2\alpha}$
(94) 13,14-dihydro-15-keto-20-ethyl-16R,S-fluoro-$PGF_{2\alpha}$ methyl ester
(95) 13,14-dihydro-15-keto-20-ethyl-11-dehydroxy-11R-methyl-$PGF_{2\alpha}$ methyl ester
(96) 13,14-dihydro-15-keto-20-ethyl-16R,S-fluoro-11-dehydroxy-11R-methyl-$PGF_{2\alpha}$ methyl ester
(97) 13, 14-dihydro-15-keto-$PGF_{2\alpha}$

Examples of substituents present in preferred compounds are as follows: Substituents on the carbon atom at 3-, 17- and/or 19-position include lower alkyl, for example, $C_{1-4}$ alkyl, especially methyl and ethyl. Substituents on the carbon atom at 16-position include lower alkyl such as methyl and ethyl, hydroxy and halogen atom such as chlorine and fluorine. Substituents on the carbon atom at 20-position include saturated and unsaturated lower alkyl such as $C_{1-4}$ alkyl, lower alkoxy such as $C_{1-4}$ alkoxy and lower alkoxy (lower) alkyl such as $C_{1-4}$ alkoxy-$C_{1-4}$ alkyl. Prostaglandins having hydroxy substituent on the carbon atom at 9- and/or 11-position include PGDs, PGEs and PGFs, stereochemistry of which at 9- and/or 11-carbon atom being alpha, beta or a mixture thereof.

Within the above exemplified compounds, 13,14-dihydro-15-keto-PGDs can be prepared according to the process described in EPA281239, 13,14-dihydro-15-keto-PGEs in EPA284180 and 13,14-dihydro-15-keto-PGFs in EPA289349 or European Patent Application No. 88308299.2 (EP-A-0 308 135), and other compounds can be prepared referring to the above processes.

The compounds of the formula(I) wherein Z is a derivative of carboxy group can be prepared by reacting the corresponding free carboxylic acid or a reactive derivative thereof with an alcohol, amine or a reactive derivative at the hydroxy or amino group thereof. The reactive derivatives at the carboxy group include acid halides, acid anhydrides, activated esters and activated amides. As the acid halides, acid chloride is most commonly used. The acid anhydrides include symmetric anhydride and mixed anhydride. Examples of the latter are dialkyl phosphoric acid mixed anhydride, dialkyl phosphorous acid mixed anhydride, alkyl carbonic acid mixed anhydride and aliphatic acid (e.g. pivalic acid and trichloroacetic acid.) mixed anhydride. The activated ester include aliphatic ester such as methyl ester, ethyl ester and cyanomethyl ester, aromatic ester such as p-nitrophenyl ester, esters with N-hydroxy compounds such as N-hydroxysuccinimide. The activated amides include amides with imidazole, dimethylimidazole and triazole. The reactive derivatives at the hydroxy group include halide and sulfonic acid (e.g. methanesulfonic acid and toluenesulfonic acid). The reactive derivatives at the amino group include Schiff's base with aldehydes (e.g. acetaldehyde, isopentanal and benzaldehyde), reaction products with silyl compounds (e.g. trimethylsilyl chloride and trimethylsilyl acetamide.) and reactive products with phosphorus compounds (e.g. phosphorus trichloride and phosphorus oxychloride.).

When a free carboxylic acid is to be used, the reaction is advantageously carried out in the presence of a condensing agent. Examples of the condensing agents are N,N-dicyclohexylcarbodimide, N-cyclohexyl-N'-morpholinoethylcarbodiimide, N,N'-diisopropylcarbodiimide, N-ethylbenzisoxazolium salt, 2-chloro-1-methylpyridinium salt, N,N'-carbonyldiimidazole, phosphorus trichloride and phosphorus oxychloride. The reaction is

usually carried out in a solvent. Examples of the solvents are dioxane, methylene chloride, tetrahydrofuran, dimethylformamide, pyridine, benzene, toluene and xylene.

The etherified cyclodextrins used according to the present invention include lower-alkylated cyclodextrins such as methyl cyclodextrin, ethylcyclodextrin, propylcyclodextrin, dimethylcyclodextrin and trimethylcyclodextrin., lower-alkenylated cyclodextrins, hydroxy-lower-alkylated cyclodextrins such as hydroxyethylcyclodextrin and hydroxypropylcyclodextrin., lower-alkoxy-lower-alkylated cyclodextrines, aralkylated cyclodextrins, such as benzylcyclodextrin, halo-lower-alkylated cyclodextrins such as chloroethylcyclodextrin., and cyclodextrin-epichlorohydrin-copolymers. These are known or can be prepared by a process analogous to that for the known compounds.

The term "intimate mixture" used herein refers to a mixture wherein two or more substances are minutely mixed and dispersed as in a solid solution and wherein constituent substances cannot be visually distinguished. It is to be appreciated that the intimate mixture include a mixture wherein the mixed constituents form an adduct or inclusion compound.

Said intimate mixture can be prepared, for example, by contacting a 13,14-dihydro-15-keto-PGs with an etherified cyclodextrine in a solvent or a solvent system in which at least one of them is at least partly soluble and then removing said solvent or solvent system. Preferred solvents or solvent systems are those in which both the constituents are completely soluble. Examples of preferred solvents and solvent systems are water and hydrophilic organic solvents such as methanol, ethanol, dioxane and acetone, as well as a mixture thereof. Dissolution can be carried out at or below room temperature or with moderate heating. The removal of the solvent can preferablely be effected by dislillation under reduced pressure or lyophilization. Resulting residue can conveniently be pulverized. The etherized cyclodextrin is suitably used in an amount more than 5 times, preferably 5 or 7 to 200 times and more preferably 5 or 8 to 100 times the amount of the 13,14-dihydro-15-keto-PGs.

The thus obtained powders can be converted into conventional solid or liquid preparations which are suitable for peroral or parenteral administration (e.g. intravenou. intraarterial, subcutaneous, intramuscular, intravaginal and intrauterine.) such as tablets, powders, granules, readily soluble solid and suspensions. The preparation may be prepared by conventional process using carriers or diluents such as starch, lactose, dextrin, mannitol water, and lubricant, humectant, perfume, preservative and colourant. Aqueous solution may be prepared by directly dissolving the two ingredients and without passing through the powders.

For the above preparations, the amount contained therein of the 13,14-dihydro-15-keto-PGs will of course vary depending the treatment desired in obstetric-gynaecological, cardiovascular, gastrointestinal and ophthalmological regions, and further on age, condition and severity of illness of the patient. In general, satisfactory results are obtainable in administration at a dosage of 0.005 - 500 mg.

A more complete understanding of the present invention can be obtained by reference to the following Examples which are provided herein for purposes of illustration only, and are not intended to limit the scope of the invention.

The following abbreviations are used in Examples.

CD: Cyclodextrine

Compound A: 13,14-Dihydro-15-keto-$PGE_2$ ethyl ester
Compound B: 13,14-Dihydro-15-keto-19-methyl-$PGE_2$ ethyl ester
Compound C: 13,14-Dihydro-6,15-diketo-$PGE_1$ ethyl ester
Compound D: 13,14-Dihydro-6,15-diketo-19-methyl-$PGE_1$ ethyl ester
Compound E: 13,14-Dihydro-6,15-diketo-11-dehydroxy-11R-methyl-$PGE_1$ ethyl ester
Compound F: 13,14-Dihydro-15-keto-16R,S-fluoro-$PGE_2$
Compound G: 13,14-Dihydro-15-keto-20-ethyl-$PGF_{2\alpha}$ isopropyl ester

Example 1

(Dimethyl-α-CD: Compound D=40:1)

An aliquot (4ml) of an aqueous solution (Solution A) containing 100mg/ml of dimethyl-α-CD was added to 10mg of compound D. The resulted mixture was stirred at room temperature until it was homogeneous and lyophilized to give a white solid in which both the components were intimately mixed.

Comparative Example 1

(α-CD: Compound D=40:1)

An aliquot (4ml) of an aqueous solution (Solution X) containing 100mg/ml of α-CD was added to 10mg of

compound D. The resulted mixture was stirred at room temperature but did not give a homogeneous solution and formed distinguishable solid and liquid phases.

Example 2

(Dimethyl-α-CD: Compound D=20:1)

The procedure in Example 1 was repeated using 2ml in place of 4ml of Solution A. An analogous white solid was obtained.

Comparative Example 2

(α-CD: Compound D=20:1)

The procedure in Comparative Example 1 was repeated using 2ml in place of 4ml of Solution X. An analogous result was obtained.

Example 3

(Dimethyl-α-CD: Compound D=10:1)

The procedure in Example 1 was repeated using 1ml in place of 4ml of Solution A. An analogous white solid was obtained.

Comparative Example 3

(α-CD: Compound D=10:1)

The procedure in Comparative Example 1 was repeated using 1ml in place of 4ml of Solution X. An analogous result was obtained.

Example 4

(Dimethyl-β-CD: Compound D=20:1)

The procedure in Example 2 was repeated using a solution (Solution B) containing 100mg/ml of dimethyl-β-CD in place of Solution A. An analogous white solid was obtained.

Example 5

(Dimethyl-β-CD: Compound D=10:1)

The procedure in Example 4 was repeated using 1ml in place of 2ml of Solution B. An analogous white solid was obtained.

Comparative Example 4

(β-CD: Compound D=13.5:1)

The procedure in Comparative Example 1 was repeated using 10ml of a solution (Solution Y) containing 13.5mg/ml of β-CD in place of Solution X. An analogous result was obtained.

Comparative Example 5

(γ-CD: Compound D=30:1)

The procedure in Comparative Example 1 was repeated using 1.25ml of a solution ( Solution Z) containing 240mg/ml of γ-CD. An analogous result was obtained.

Example 6

(Hydroxypropyl-β-CD: Compound D=40:1)

The procedure in Example 1 was repeated using 4ml of solution (Solution C) containing 100mg/ml of hydroxypropyl-β-CD in place of Solution A. An analogous white solid was obtained.

Example 7

(Dimethyl-α-CD: Compound D=30:1)

The procedure in Example 1 was repeated using 3ml of Solution A and a compound selected from Compound A, B, C, E and F. An analogous white solid was obtained.

Comparative Example 6

(α-CD: Compound D=30:1)

The procedure in Comparative Example 1 was repeated using 3ml of Solution X and a compound selected from Compound A, B, C, E and F. An analogous result was obtained.

Comparative Example 7 (Known Technique)

(α-CD: $PGE_2$=30:1)

The procedure in Comparative Example 1 was repeated using 3ml of Solution X and 10mg of $PGE_2$ to give a homogeneous solution, which, on lyophilization, gave a white solid.

Example 8

(Dimethyl-α-CD: Compound G=20:1)

The procedure in Example 1 was repeated using 2ml of Solution A and Compound G. An analogous white solid was obtained.

Example 9

(Heat Stability)

Each of the white solids obtained in Examples 1-5 was tested for heat stability by heating in a thermostatic oven held at 60°C. Residual rate of Compound D are shown in Table 1.

Assay protocol for residual rate was as follows: The sample heated for a predetermined period of time was dissolved in aliquot of distilled water. A prescribed amount of the solution was assayed by High Performance Liquid Chromatography (HPLC) (Instrument: Hitachi 655A) under the following conditions.

Detection Wavelength 210nm
Mobile Phase $CH_3CN:H_2O$=1:1
Flow Rate 1ml/min
Column Package High Pore RP-18,
Particle Size: 10μm, 4x250mm

Table 1

| Sample | Residual Rate of Compound D(%) | |
|---|---|---|
| | 60°C, 5 Days | 60°C, 14 Days |
| Example 1 | 100 | 99 |
| " 2 | 100 | 99 |
| " 3 | 100 | 99 |
| " 4 | 99 | 98 |
| " 5 | 98 | 96 |
| Compound D (as such) | 86 | 56 |

The above results demonstrate that the stability of Compound D was improved by etherized cyclodextrins.

Example 10

(Stability in Aqueous Solution)

Each of the white solids obtained in Examples 3 and 5 was dissolved in distilled water to form a solution containing 44mg/ml (4mg Compound D/ml). The solution was tested for stability by placing in a stoppered vial for a predetermined period of time at room temperature. Residual rate of Compound D are shown in Table 2.

Assay protocal for residual rate using internal standard HPLC was as follows:

The sample (aqueous solution) left for a predetermined period of time at room temperature was combined with an acetonitrile solution containing an amount of internal standard. After mixing thoroughly, an aliquot of the solution was assayed by HPLC (instrument:Hitachi 655A) under the following conditions.

Detection Wavelength 282nm
Mobile Phase $CH_3CN:H_2O=3:2$
Flow Rate 1ml/min
Column Package
High Pore RP-18,
Particle Size: 10μm,
4x250mm

Table 2

| Sample | Residual Rate of Compound D(%) | | | |
|---|---|---|---|---|
| | Room Temperature, Days | | | |
| | 1 | 2 | 3 | 4 |
| Example 3 | 98.4 | 96.8 | 95.9 | 89.2 |
| " 5 | 99.5 | 96.6 | 96.0 | 91.3 |

The above results demonstrate that the stability of Compound D was improved by etherized cyclodextrins.

## Claims

### Claims for the following Contracting States :AT, BE, CH, DE, GB, FR, IT, LI, LU, NL, SE

1. A stabilized 13,14-dihydro-15-ketoprostaglandin composition comprising an intimate mixture of
a) a therapeutically effective amount of at least one compound selected from 13,14-dihydro-15-ketoprostaglandins and
b) at least one compound selected from pharmaceutically acceptable etherized cyclodextrins
characterised in that the ratio by weight of component (a) to component (b) is between 1:5 and 1:200.

2. The composition of claim 1, in which said 13,14-dihydro-15-ketoprostaglandins are represented by the formula:

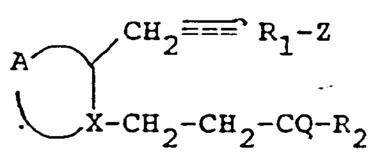

(I)

wherein the group:

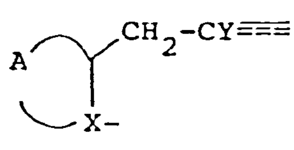

is a radical selected from following formulae:

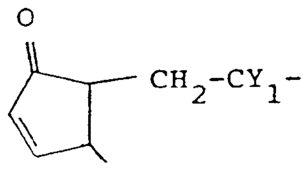

(i)

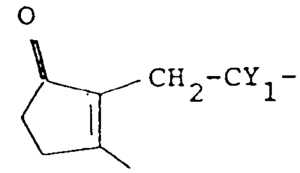

(ii)

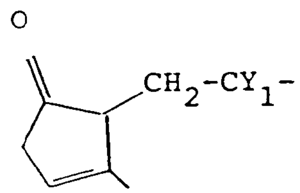

(iii)

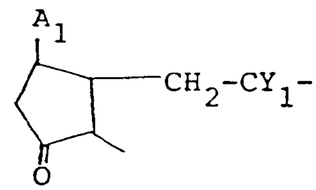

(iv)

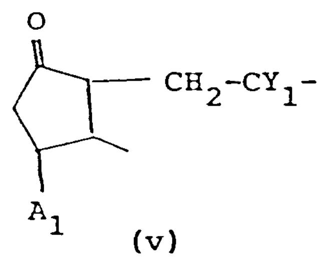

(v)

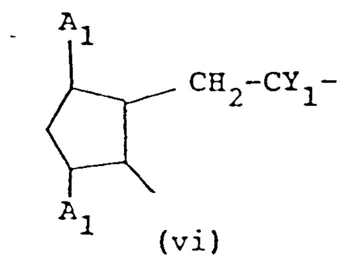

(vi)

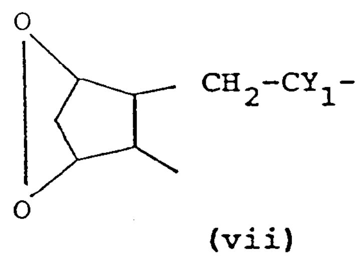

(vii)

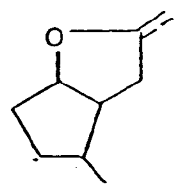

(viii)

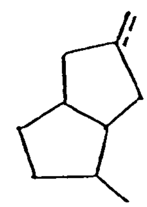

(ix)

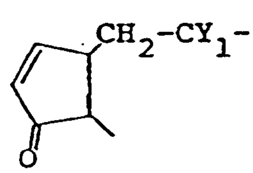

(x)

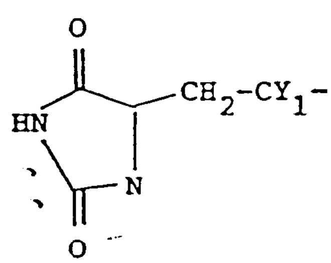

(xi)

$A_1$ is hydroxy, lower alkyl or hydroxy ($C_{1-6}$) alkyl,

$Y_1$ is 0, 1 or 2 hydrogen atoms or oxo,

Z is a hydroxymethylcarbonyl group, a carboxyl group or a functional derivative of a carboxyl group,

$R_1$ is saturated or unsaturated $C_{1-6}$ aliphatic hydrocarbon residue,

$R_2$ is saturated or unsaturated $C_{1-9}$ aliphatic hydrocarbon residue which is unsubstituted or substituted with at least one substituent selected from hydroxy, halo, $C_{1-6}$ alkoxyphenyl and phenoxy,

the symbol of a line and a dotted line is single bond or double bond, and the symbol of a line and two dotted line is single bond, double bond or triple bond,

3. The composition of claim 1, in which said etherized cyclodextrins are cyclodextrin ($C_{1-6}$) alkyl ethers, cyclodextrin ($C_{1-6}$) alkenyl ethers, cyclodextrin-hydroxy-($C_{1-6}$) alkyl ethers, cyclodextrin ($C_{1-6}$) alkoxy ($C_{1-6}$) alkyl

ethers, cyclodextrin-monocyclic aromatic ($C_{1-6}$) alkyl ethers, cyclodextrin-halo ($C_{1-6}$) alkyl ethers, or cyclodextrin-epichlorhydrin-copolymers.

4. The composition of claim 1, in which the ratio of component (a) to component (b) is between 1:7 and 1:200.

5. The composition of claim 1, in which the ratio is between 1:5 and 1:100.

6. The composition of claim 4, in which the ratio is between 1:8 and 1:100.

7. The composition of claim 4, in which the ratio is between 1:10 and 1:40.

8. A method of preparing a stabilized 13,14-dihydro-15-ketoprostaglandin composition which comprises intimately mixing

(a) a therapeutically effective amount of at least one compound selected from 13,14-dihydro-15-ketoprostaglandins and

(b) at least one compound selected from pharmaceutically acceptable etherized cyclodextrins characterised in that the ratio by weight of component (a) to component (b) is between 1:5 and 1:200.

9. A method as claimed in claim 8 wherein the (a) 13,14-dihydro-15-ketoprostaglandin is contacted with the (b) etherized cyclodextrin in a solvent or solvent system selected from water, methanol, ethanol, dioxane, acetone and mixtures thereof.

**Claims for the following Contracting States : ES, GR**

1. A method of preparing a stabilized 13,14-dihydro-15-ketoprostaglandin composition which comprises intimately mixing

(a) a therapeutically effective amount of at least one compound selected from 13,14-dihydro-15-ketoprostaglandins and

(b) at least one compound selected from pharmaceutically acceptable etherized cyclodextrins characterised in that the ratio by weight of component (a) to component (b) is between 1:5 and 1:200.

2. A method as claimed in claim 1 wherein the (a) 13,14-dihydro-15-ketoprostaglandin is contacted with the (b) etherized cyclodextrin in a solvent or solvent system selected from water, methanol, ethanol dioxane, acetone, and mixtures thereof.

3. A method as claimed in claim 1, in which said 13,14-dihydro-15-ketoprostaglandins are represented by the formula:

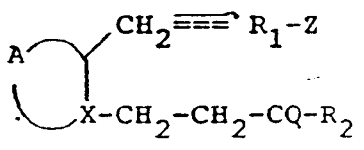

(I)

wherein the group:

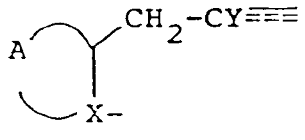

is a radical selected from the following formulae:

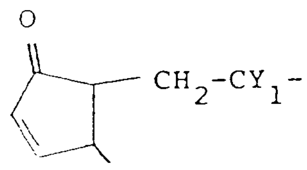

(i)

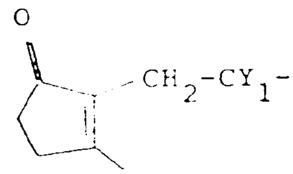

(ii)

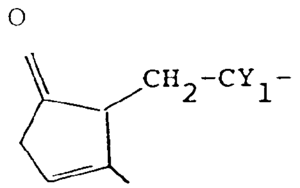

(iii)

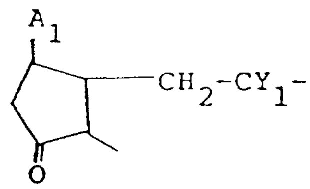

(iv)

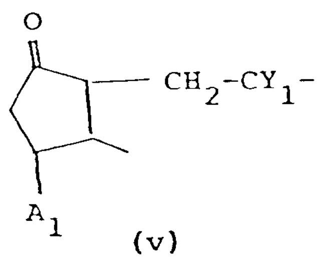

(v)

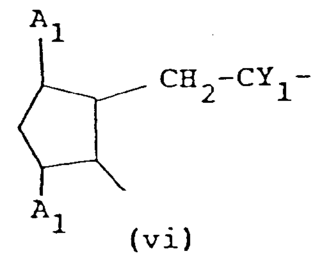

(vi)

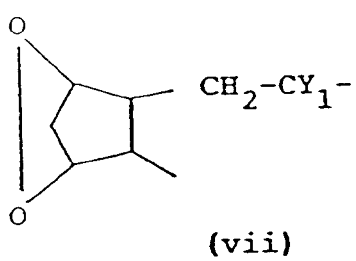

(vii)

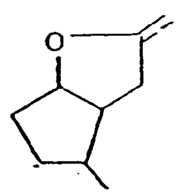

(viii)

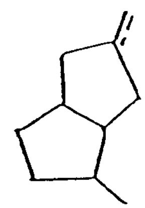

(ix)

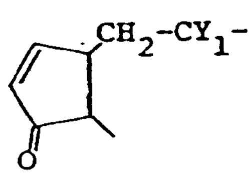

(x)

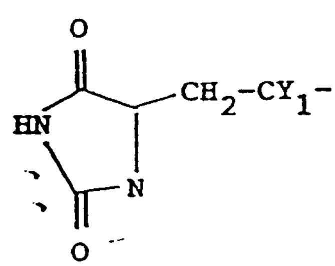

(xi)

$A_1$ is hydroxy, lower alkyl or hydroxy $(C_{1-6})$ alkyl,

$Y_1$ is 0, 1 or 2 hydrogen atoms or oxo,

Z is a hydroxymethylcarbonyl group, a carboxyl group or a functional derivative of a carboxyl group,

$R_1$ is saturated or unsaturated $C_{1-6}$ aliphatic hydrocarbon residue,

$R_2$ is saturated or unsaturated $C_{1-9}$ aliphatic hydrocarbon residue

which is unsubstituted or substituted with at least one substituent selected from hydroxy, halo, $C_{1-6}$ alkoxyphenyl and phenoxy,

the symbol of a line and a dotted line is single bond or double bond, and the symbol of a line and two dotted line is single bond, double bond or triple bond,

4. A method as claimed in claim 1, in which said etherized cyclodextrins are cyclodextrin $(C_{1-6})$ alkyl ethers,

cyclodextrin ($C_{1-6}$) alkenyl ethers, cyclodextrin-hydroxy-($C_{1-6}$) alkyl ethers, cyclodextrin ($C_{1-6}$) alkoxy ($C_{1-6}$) alkyl ethers, cyclodextrin-monocyclic aromatic ($C_{1-6}$) alkyl ethers, cyclodextrin-halo ($C_{1-6}$) alkyl ethers, or cyclodextrin-epichlorhydrin-copolymers.

5. A method as claimed in claim 1, in which the ratio of component (a) to component (b) is between 1:7 and 1:200.

6. A method as claimed in claim 1, in which the ratio is between 1:5 and 1:100.

7. A method as claimed in claim 6, in which the ratio is between 1:8 and 1:100.

8. A method as claimed in claim 7, in which the ratio is between 1:10 and 1:40.

**Patentansprüche**

**Patentansprüche für folgende Vertragsstaaten : AT, BE, CH, DE, GB FR, IT, LI, LU, NL, SE**

1. Stabilisierte 13,14-Dihydro-15-ketoprostaglandin-Zusammensetzung, die ein inniges Gemisch aus

(a) einer therapeutisch wirksamen Menge von mindestens einer Verbindung, ausgewählt unter 13,14-Dihydro-15-ketoprostaglandinen und

(b) zumindest einer Verbindung, ausgewählt unter pharmazeutisch annehmbaren verätherten Cyclodextrinen

enthält, dadurch **gekennzeichnet**, daß das Gewichtsverhältnis der Komponente (a) zu Komponente (b) zwischen 1:5 und 1:200 liegt.

2. Zusammensetzung nach Anspruch 1, **dadurch gekennzeichnet**, daß die 13,14-Dihydro-15-ketoprostaglandine durch die Formel:

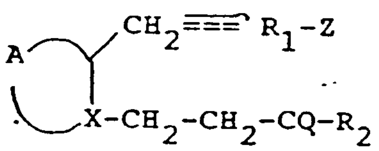

(I)

dargestellt werden, worin die Gruppe

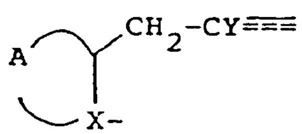

eine Gruppe ist, ausgewählt unter den folgenden Formeln:

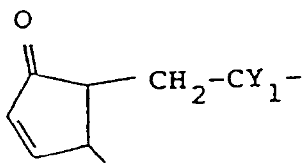

(i)

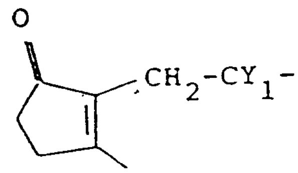

(ii)

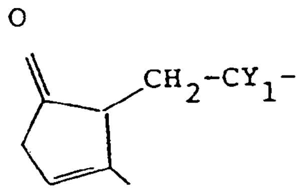

(iii)

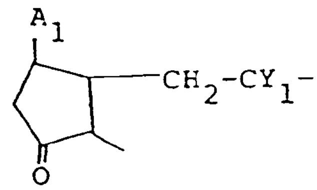

(iv)

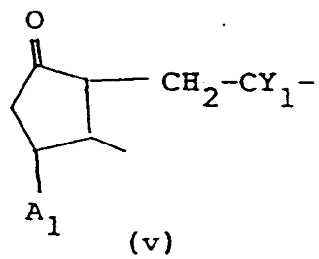

(v)

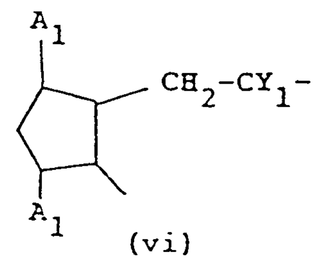

(vi)

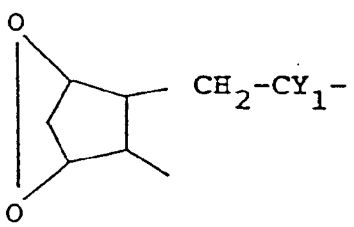

(vii)

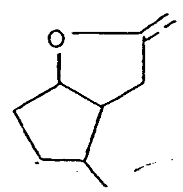

(viii)

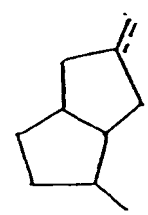

(ix)

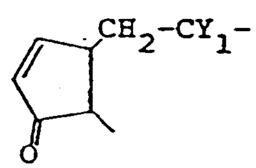

(x)

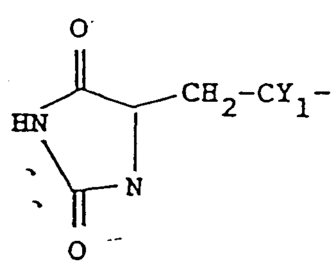

(xi)

worin

$A_1$ Hydroxy, Niedrigalkyl oder Hydroxy($C_{1-6}$)-alkyl bedeutet,

$Y_1$ 0, 1 oder 2 Wasserstoffatome oder Oxo bedeutet,

Z eine Hydroxymethylcarbonylgruppe, eine Carboxylgruppe oder ein funktionelles Derivat einer Carboxylgruppe bedeutet,

$R_1$ eine gesättigte oder ungesättigte $C_{1-6}$ aliphatische Kohlenwasserstoffgruppe bedeutet,

$R_2$ eine gesättigte oder ungesättigte $C_{1-9}$ aliphatische Kohlenwasserstoffgruppe bedeutet, welche unsubstituiert oder substituiert mit mindestens einem Substituenten, ausgewählt unter Hydroxy, Halo, $C_{1-6}$-Alkoxyphenyl und Phenoxy ist,

das Symbol einer Linie oder gestrichelten Linie eine Einfach- oder Doppelbindung bedeutet, und das Symbol einer Linie und zweigestrichelten Linie eine Einfachbindung, Doppelbindung oder Dreifachbindung bedeutet.

3. Zusammensetzung nach Anspruch 1, dadurch **gekennzeichnet**, daß die verätherten Cyclodextrine Cy-

clodextrin-($C_{1-6}$)-alkyläther, Cyclodextrin-($C_{1-6}$)-alkenyläther, Cyclodextrin-hydroxy-($C_{1-6}$)-alklyäther, Cyclodextrin-($C_{1-6}$)-alkoxy-($C_{1-6}$)-alkyläther, Cyclodextrin-monozyklisch-aromatisch-($C_{1-6}$)-alkyläther, Cyclodextrin-halo-($C_{1-6}$)-alkyläther oder Cyclodextrin-epichlorhydrin-Copolymere sind.

4. Zusammensetzung nach Anspruch 1, dadurch **gekennzeichnet**, daß das Verhältnis der Komponente (a) zur Komponente (b) zwischen 1:7 und 1:200 liegt.

5. Zusammensetzung nach Anspruch 1, dadurch **gekennzeichnet**, daß das Verhältnis zwischen 1:5 und 1:100 liegt.

6. Zusammensetzung nach Anspruch 4, dadurch **gekennzeichnet**, daß das Verhältnis zwischen 1:8 und 1:100 liegt.

7. Zusammensetzung nach Anspruch 4, dadurch **gekennzeichnet**, daß das Verhältnis zwischen 1:10 und 1:40 liegt.

8. Verfahren zur Herstellung einer stabilisierten 13,14-Dihydro-15-ketoprostaglandin-Zusammensetzung durch inniges Vermischen von

(a) einer therapeutisch wirksamen Menge von mindestens einer Verbindung, ausgewählt unter 13,14-Dihydro-15-ketoprostaglandin und

(b) mindestens einer Verbindung, ausgewählt unter pharmazeutisch annehmbaren verätherten Cyclodextrinen, dadurch **gekennzeichnet**, daß das Gewichtsverhältnis von Komponente (a) zu Komponente (b) zwischen 1:5 und 1:200 liegt.

9. Verfahren nach Anspruch 8, dadurch **gekennzeichnet**, daß (a) 13,14-Dihydro-15-ketoprostaglandin mit (b) veräthertem Cyclodextrin in einem Lösungsmittel oder in einem Lösungsmittelsystem, ausgewählt unter Wasser, Methanol, Ethanol, Dioxan, Aceton und ihren Gemischen, behandelt wird.

**Patentansprüche für folgende Vertragsstaaten : ES, GR**

1. Verfahren zur Herstellung einer stabilisierten 13,14-Dihydro-15-ketoprostaglandin-Zusammensetzung durch inniges Vermischen

(a) einer therapeutisch wirksamen Menge von mindestens einer Verbindung, ausgewählt unter 13,14-Dihydro-15-ketoprostaglandin und

(b) mindestens einer Verbindung, ausgewählt unter pharmazeutisch annehmbaren verätherten Cyclodextrinen, dadurch **gekennzeichnet**, daß das Gewichtsverhältnis von Komponente (a) zu Komponente (b) zwischen 1:5 und 1:200 liegt.

2. Verfahren nach Anspruch 1, dadurch **gekennzeichnet**, daß (a) 13,14-Dihydro-15-ketoprostaglandin mit (b) veräthertem Cyclodextrin in einem Lösungsmittel oder in einem Lösungsmittelsystem, ausgewählt unter Wasser, Methanol, Ethanol, Dioxan, Aceton und ihren Gemischen, behandelt wird.

3. Verfahren nach Anspruch 1, dadurch **gekennzeichnet**, daß die 13,14-Dihydro-15-ketoprostaglandine hergestellt werden, welche durch die Formel

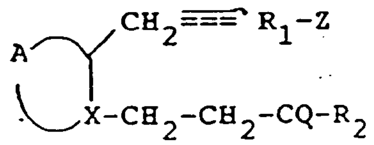

(I)

dargestellt werden, worin die Gruppe

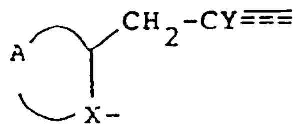

eine Gruppe ist, ausgewählt unter den folgenden Formeln

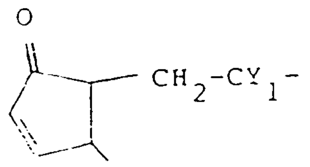

(i)

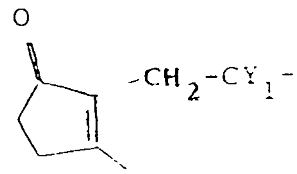

(ii)

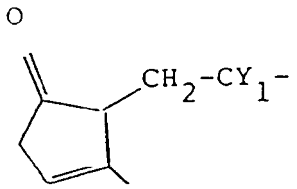

(iii)

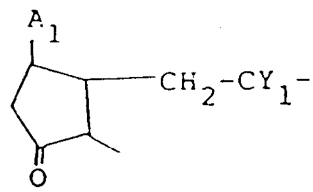

(iv)

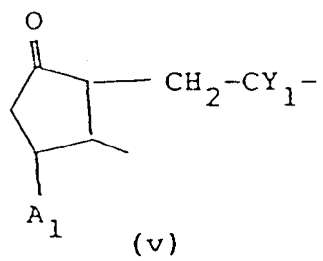

(v)

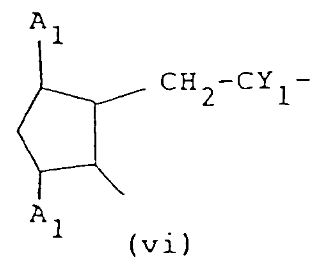

(vi)

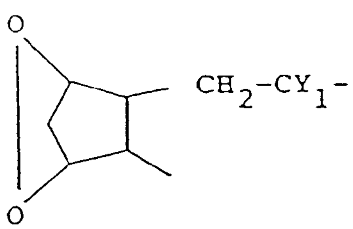

(vii)

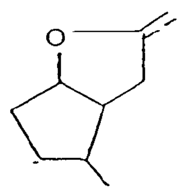

(viii)

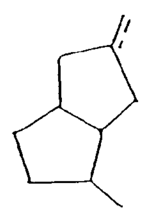

(ix)

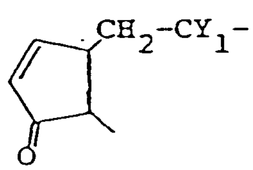

(x)

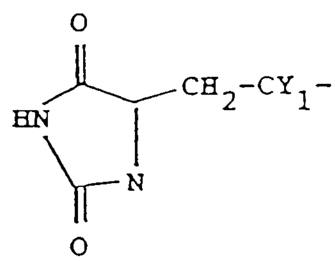

(xi)

worin

$A_1$ Hydroxy, Niedrigalkyl oder Hydroxy($C_{1-6}$)-alkyl bedeutet,

$Y_1$ 0, 1 oder 2 Wasserstoffatome oder Oxo bedeutet,

Z eine Hydroxymethylcarbonylgruppe, eine Carboxylgruppe oder ein funktionelles Derivat einer Carboxylgruppe bedeutet,

$R_1$ eine gesättigte oder ungesättigte $C_{1-6}$ aliphatische Kohlenwasserstoffgruppe bedeutet,

$R_2$ eine gesättigte oder ungesättigte $C_{1-9}$ aliphatische Kohlenwasserstoffgruppe bedeutet, welche unsubstituiert oder substituiert mit mindestens einem Substituenten, ausgewählt unter Hydroxy, Halo, $C_{1-6}$-Alkoxyphenyl und Phenoxy ist,

das Symbol einer Linie oder gestrichelten Linie eine Einfach- oder Doppelbindung bedeutet, und das Symbol

einer Linie und zweigestrichelten Linie eine Einfachbindung, Doppelbindung oder Dreifachbindung bedeutet.

4. Verfahren nach Anspruch 1, dadurch **gekennzeichnet**, daß die verätherten Cyclodextrine Cyclodextrin-($C_{1-6}$)-alkyläther, Cyclodextrin-($C_{1-6}$)-alkenyläther, Cyclodextrin-hydroxy-($C_{1-6}$)-alklyäther, Cyclodextrin-($C_{1-6}$)-alkoxy-($C_{1-6}$)-alkyläther, Cyclodextrin-monozyklisch-aromatisch-($C_{1-6}$)-alkyläther, Cyclodextrin-halo-($C_{1-6}$)-alkyläther oder Cyclodextrin-epichlorhydrin-Copolymere sind.

5. Verfahren nach Anspruch 1, dadurch **gekennzeichnet**, daß das Verhältnis der Komponente (a) zu Komponente (b) zwischen 1:7 und 1:200 liegt.

6. Verfahren nach Anspruch 1, dadurch **gekennzeichnet**, daß das Verhältnis zwischen 1:5 und 1:100 liegt.

7. Verfahren nach Anspruch 6, dadurch **gekennzeichnet**, daß das Verhältnis zwischen 1:8 und 1:100 liegt.

8. Verfahren nach Anspruch 7, dadurch **gekennzeichnet**, daß das Verhältnis zwischen 1:10 und 1:40 liegt.

## Revendications

### Revendications pour les Etats contractants suivants : AT, BE, CH, DE, GB, FR, IT, LI, LU, NL, SE

1. Composition de 13,14-dihydro-15-cétoprostaglandine stabilisée, comprenant un mélange intime

(a) d'une quantité thérapeutiquement efficace d'au moins un composé choisi parmi les 13,14-dihydro-15-cétoprostaglandines et

(b) d'au mois un composé choisi parmi des cyclodextrines éthérifiées pharmaceutiquement acceptables, caractérisée en ce que le rapport pondéral du constituant (a) au constituant (b) est compris entre 1:5 et 1:200.

2. Composition selon la revendication 1, dans laquelle ces 13,14-dihydro-15-cétoprostaglandines sont représentées par la formule :

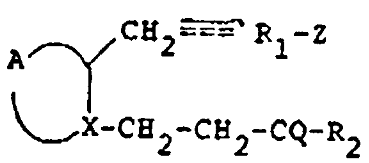

(I)

dans laquelle le groupe :

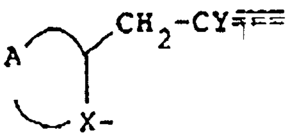

est un radical choisi parmi les formules suivantes :

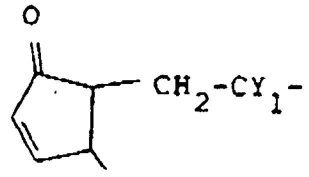

(i)

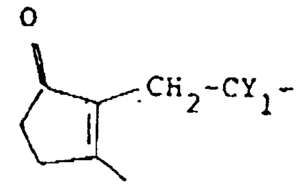

(ii)

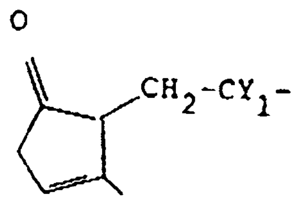

(iii)

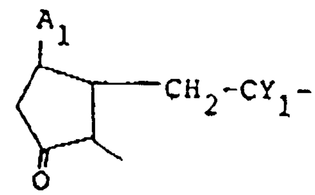

(iv)

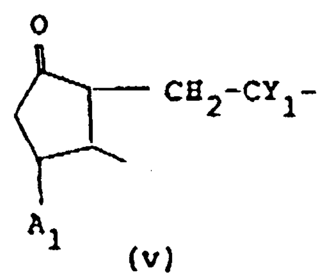

(v)

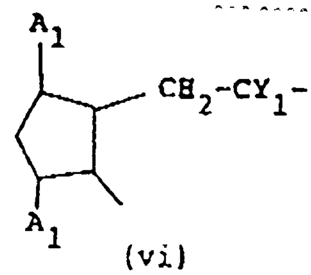

(vi)

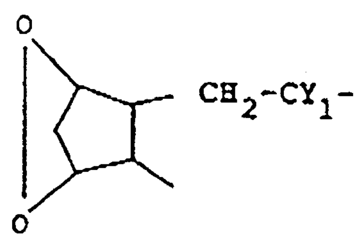

(vii)

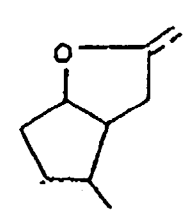

(viii)

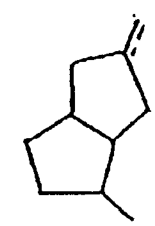

(ix)

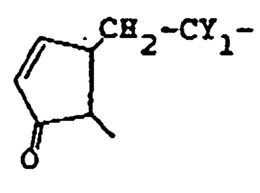

(x)

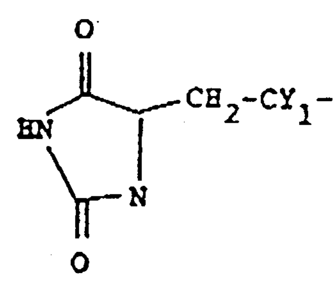

(xi)

$A_1$ est un groupe hydroxy, alkyle inférieur ou hydroxy (alkyle en $C_1$ à $C_6$),
$Y_1$ est 0, 1 ou 2 atomes d'hydrogène ou un groupe oxo,
Z est un groupe hydroxyméthylcarbonyle, un groupe carboxyle ou un dérivé fonctionnel d'un groupe carboxyle,
$R_1$ est un radical hydrocarboné aliphatique saturé ou insaturé en $C_1$ à $C_6$,
$R_2$ est un radical hydrocarboné aliphatique saturé ou insaturé en $C_1$ à $C_9$ qui est non substitué ou substitué par au moins un substituant choisi parmi le groupe hydroxy, un atome d'halogène, un groupe (alcoxy en $C_1$ à $C_6$) phényle et un groupe phénoxy,
le symbole constitué d'une ligne en trait plein et d'une ligne en trait interrompu est une simple liaison ou une double liaison, et le symbole constitué d'une ligne en trait plain et de deux lignes en trait interrompu est une

simple liaison, une double liaison ou une triple liaison.

3. Composition selon la revendication 1, dans laquelle ces cyclodextrines éthérifiées sont des éthers de cyclodextrine et d'alkyle en $C_1$ à $C_6$, des éthers de cyclodextrine et d'alcényle en $C_1$ à $C_6$, des éthers de cyclodextrine et d'hydroxyalkyle en $C_1$ à $C_6$, des éthers de cyclodextrine et d'alcoxy en $C_1$ à $C_6$ (alkyle en $C_1$ à $C_6$), des éthers de cyclodextrine et d'aromatiques monocycliques (alkyle en $C_1$ à $C_6$), des éthers de cyclodextrine et d'haloalkyle en $C_1$ à $C_6$, ou des copolymères cyclodextrine-épichlorhydrine.

4. Composition selon la revendication 1, dans laquelle le rapport du constituant (a) au constituant (b) est compris 1:7 et 1:200.

5. Composition selon la revendication 1, dans laquelle le rapport est compris entre 1:5 et 1:100.

6. Composition selon la revendication 4, dans laquelle le rapport est compris entre 1:8 et 1:100.

7. Composition selon la revendication 4, dans laquelle le rapport est compris entre 1:10 et 1:40.

8. Procédé de préparation d'une composition de 13,14-dihydro-15-cétoprostaglandine stabilisée, qui comprend le fait de mélanger intimement

(a) une quantité thérapeutiquement efficace d'au moins un composé choisi parmi des 13,14-dihydro-15-cétoprostaglandines et

(b) au mois un composé choisi parmi des cyclodextrines éthérifiées pharmaceutiquement acceptables, caractérisé en ce que le rapport pondéral du constituant (a) au constituant (b) est compris 1:5 et 1:200.

9. Procédé selon la revendication 8, dans lequel la 13,14-dihydro-15-cétoprostaglandine (a) est mise en contact avec la cyclodextrine éthérifiée (b) dans un solvant ou un système de solvant choisi parmi l'eau, le méthanol, l'éthanol, le dioxane, l'acétone et leurs mélanges.

**Revendications pour les Etats contractants suivants : ES, GR**

1. Procédé de préparation d'une composition de 13,14-dihydro-15-cétoprostaglandine stabilisée, qui comprend le fait de mélanger intimement

a) une quantité thérapeutiquement efficace d'au moins un composé choisi parmi les 13,14-dihydro-15-cétoprostaglandines et

b) au mois un composé choisi parmi les cyclodextrines éthérifiées pharmaceutiquement acceptables, caractérisé en ce que le rapport pondéral du constituant (a) au constituant (b) est compris entre 1:5 et 1:200.

2. Procédé selon la revendication 1, dans lequel la 13,14-dihydro-15-cétoprostaglandine (a) est mise en contact avec la cyclodextrine ethérifiée (b) dans un solvant ou un système de solvant choisi parmi l'eau, le méthanol, l'éthanol, le dioxane, l'acétone et leurs mélanges.

3. Procédé selon la revendication 1, dans lequel ces 13,14-dihydro-15-cétoprostaglandines sont représentées par la formule :

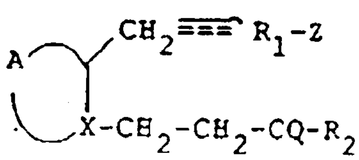

(I)

dans laquelle le groupe :

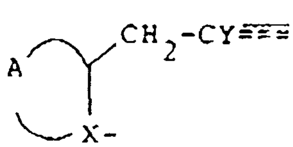

est un radical choisi parmi les formules suivantes :

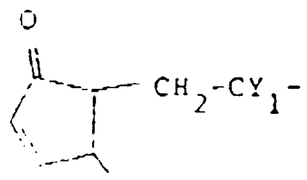

(i)

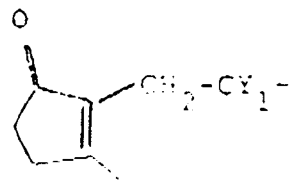

(ii)

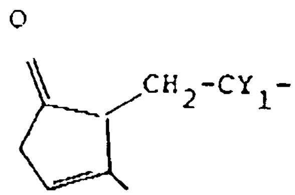

(iii)

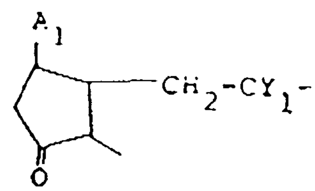

(iv)

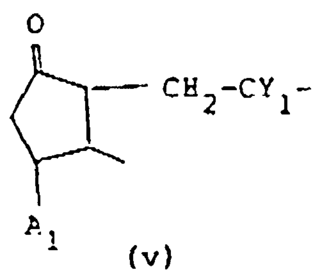

(v)

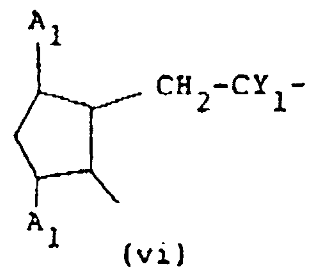

(vi)

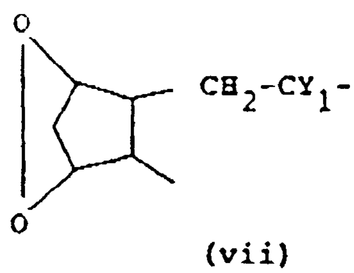

(vii)

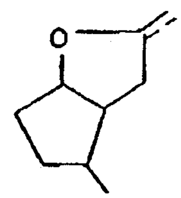

(viii)

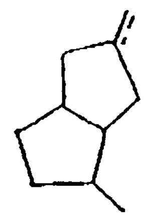

(ix)

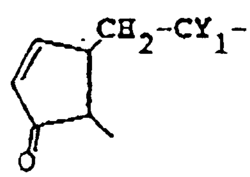

(x)

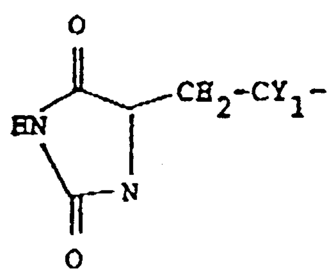

(xi)

$A_1$ est un groupe hydroxy, alkyle inférieur ou hydroxy (alkyle en $C_1$ à $C_6$),
$Y_1$ est 0, 1 ou 2 atomes d'hydrogène ou un groupe oxo,
Z est un groupe hydroxyméthylcarbonyle, un groupe carboxyle ou un dérivé fonctionnel d'un groupe carboxyle,
$R_1$ est un radical hydrocarboné aliphatique saturé ou insaturé en $C_1$ à $C_6$,
$R_2$ est un radical hydrocarboné aliphatique saturé ou insaturé en $C_1$ à $C_9$ qui est non substitué ou substitué par au moins un substituant choisi parmi le groupe hydroxy, un atome d'halogène, un groupe (alcoxy en $C_1$ à $C_6$)phényle et un groupe phénoxy,
le symbole constitué d'une ligne en trait plein et d'une ligne en trait interrompu est une simple liaison ou une double liaison, et le symbole constitué d'une ligne en trait plein et de deux lignes en trait interrompu est une simple liaison, une double liaison ou une triple liaison.

4. Procédé selon la revendication 1, dans lequel ces cyclodextrines éthérifiées sont des éthers de cyclodextrine et d'alkyle en $C_1$ à $C_6$, des éthers de cyclodextrine et d'alcényle en $C_1$ à $C_6$, des éthers de cyclodextrine et d'hydroxyalkyle en $C_1$ à $C_6$, des éthers de cyclodextrine et d'alcoxy en $C_1$ à $C_6$ (alkyle en $C_1$ à $C_6$), des éthers de cyclodextrine et d'aromatiques monocycliques (alkyle en $C_1$ à $C_6$), des éthers de cyclodextrine et d'haloalkyle en $C_1$ à $C_6$, ou des copolymères cyclodextrine-épichlorhydrine.

5. Procédé selon la revendication 1, dans lequel le rapport du constituant (a) au constituant (b) est compris entre 1:7 et 1:200.

6. Procédé selon la revendication 1, dans lequel le rapport est compris entre 1:5 et 1:100.

7. Procédé selon la revendication 6, dans lequel le rapport est compris entre 1:8 et 1:100.

8. Procédé selon la revendication 7, dans lequel le rapport est compris entre 1:10 et 1:40.